Europäisches Patentamt

(19) European Patent Office

Office européen des brevets

(11) Publication number: **0 244 262**

A1

## (12) EUROPEAN PATENT APPLICATION

(21) Application number: **87303925.9**

(22) Date of filing: **30.04.87**

(51) Int. Cl.³: **C 12 N 9/02**
**C 12 P 7/00**

(30) Priority: **01.05.86 GB 8610670**

(43) Date of publication of application:
**04.11.87 Bulletin 87/45**

(84) Designated Contracting States:
**BE DE ES FR GB IT NL**

(71) Applicant: **The British Petroleum Company p.l.c.**
**Britannic House Moor Lane**
**London EC2Y 9BU(GB)**

(72) Inventor: **Broda, Paul Martin Andrew**
**21 Elm Road**
**Manchester M20 0XD(GB)**

(72) Inventor: **Mason, John Clark**
**Appartment 3, 151 Palatine Road**
**Didsbury, Manchester M20 9YA(GB)**

(72) Inventor: **Zimmermann, Wolfgang Karl**
**Flat 3, 146 Slade Lane**
**Manchester M19 2AQ(GB)**

(74) Representative: **Ryan, Edward Terrence et al,**
**BP INTERNATIONAL LIMITED, Patents Division Chertsey**
**Road**
**Sunbury-on-Thames Middlesex, TW16 7LN(GB)**

(54) **Decomposition of lignocellulose.**

(57) Lignocellulose is decomposed by a cell-free aqueous medium contacting ane enzyme derived from an actinomycete bacterium e.g. *Streptomyces cyaneus*.

EP 0 244 262 A1

Croydon Printing Company Ltd.

DECOMPOSITION OF LIGNOCELLULOSE

The present invention relates to the decomposition of lignocellulose and to enzymes suitable for use in the process.

Many plant materials contain lignocellulose in which the polysaccharide cellulose occurs in association with lignin, which is a non-polysaccharide material. Cellulose is a useful material for many purposes e.g. in the preparation of paper or in the preparation of textile fibres. Cellulose can also be broken down to give products which are more easily assimilated by a wider range of animals. However for these various uses it is necessary to separate the cellulose from the lignin.

The existence of an extracellular enzyme of the white rot fungus Phanerochaete chrysosporium which attacks lignin was disclosed in M. Tien and T.K. Kirk, Science 221 661-663 1983: "Lignin-degrading enzyme from the hymenomycete Phanerochaete chrysosporium Burds". They also showed that it carries a haem group, and that it requires hydrogen peroxide for its action. It is a peroxidase (Harvey et al., FEBS Letters 183 13-16, 1985: "Single-electron transfer processes and the reaction mechanism of enzymic degradation of lignin"). It has been proposed to develop pure ligninase preparations for treating pulp, e.g. for bleaching. Such work is being done by Kirk together with Repligen, Inc. of Cambridge, Mass., USA who have described a number of ligninases from P. chrysosporium (T.K. Kirk, S. Croan, M. Tien, K. Murtagh and R.L. Farrell, Enzyme Microb. Technol. 8 27-32 (1986): "Production of multiple ligninases by Phanerochaete chrysosporium: effect of

1

selected growth conditions and use of a mutant strain"), and are seeking to introduce the corresponding genes into expression systems in other organisms.

It has been disclosed that certain actinomycete bacteria can solublise lignin in lignocellulose (e.g. Pometto and Crawford, Appl. Env. Microbiol. 51 171-179 (1986): "Catabolic fate of Streptomyces viridosporus T7A-produced acid precipitable polymeric lignin upon incubation with ligninolytic Streptomyces species and Phanerochaete chrysosporium; McCarthy and Broda, J. Gen. Micro. 130 2905-2913 (1984): "Screening for lignin-degrading actinomycetes and characterisation of their activity against $^{14}C$-lignin-labelled wheat lignocellulose").

We have now found that it is possible to obtain extracellular enzymes effective for lignocellulose decomposition from such organisms.

According to the present invention the process for the decomposition of lignocellulose comprises bringing the lignocellulose into contact with an aqueous medium containing cell-free enzyme having lignin solubilising activity derived from an actinomycete bacterium.

This enzyme in its crude cell-free form has a very high activity for solubilising the lignin from lignocellulose.

The ability to obtain an enzyme free of bacterial cells is highly desirable. If the decomposition of lignins is carried out in the presence of bacterial cells there is a risk that metabolites released by the cell either while alive or as a result of the death and breakdown of the cell will have an adverse effect on the ligninase. More importantly the ability to obtain cell-free enzyme facilities the production of an enzyme preparation free from cellulases, hemicellulases and proteases. Cellulases and hemicellulases will cause degradation of potentially valuable materials, and proteases will inactivate the lignin-solubilising enzyme.

The enzyme may be used as a crude (but cell-free) extract. For maximum activity a purified enzyme is preferred.

The present invention also provides a novel cell-free enzyme preparation suitable for use in lignin decomposition and substantially free of cellulases and xylanases, which enzyme preparation has the following properties:

(a)  apparent molecular weight of 20000 during gel filtration under non-denaturing conditions which preserve biological activity,

(b)  an isoelectric point in the pH range 5 to 6.5.

Until now it has not been realised that actinomycete bacteria are capable of breaking down lignin by a mechanism which uses an extracellular enzyme which does not require hydrogen peroxide as a cofactor.

The Production of the Enzyme

The preferred source of the enzyme preparation are actinomycete bacteria, more particularly Streptomyces cyaneus strain MT813 and Thermomonospora mesophila strain DSM 4308.  The strain numbers identify strains held in the culture collection of the University of Manchester Institute of Science and Technology, PO Box 88, Manchester M60 1QD.  The strains identified as DSM 43087 and MT813 have been deposited under the provisions of the Budapest Treaty at the National Collections of Industrial and Marine Bacteria Ltd, Torry Research Station, Aberdeen, under the NCIB numbers 12382 and 12383 respectively.

In order to produce a good yield of the cell-free enzyme from the actinomycete bacterium it is necessary to grow the bacterium in a liquid medium which contains lignocellulose, preferably in finely divided form.  Thus the bacterium may be grown for example in a medium containing ball-milled barley straw, which may for example have a particle size in the range 0.5 to 5 micrometres.

The optimum growth conditions may vary depending on the individual bacterium used to produce the lignin-solubilising enzyme.  The pH is preferably in the range 6 to 8.  The temperature is preferably in the range 30° to 45°C, more preferably 30° to 37°C.

The optimum growth period for producing the maximum amount of lignin-solubilising enzyme may also depend on the bacterium used. Thus for S. cyaneus the maximum yield of lignocellulase may be obtained after 2 to 4 days of growth.

The cell-free enzyme may be obtained by filtering the liquid medium containing suspended bacterial cells through a filter through which bacteria cannot pass.

It may be desirable to add a precipitating agent, e.g. ammonium sulphate, to the cell-free liquid in order to precipitate and partially purify extracellular protein.

In order to obtain an enzyme of increased purity, capable of solubilising lignin from lignocellulose, for example substantially free of other types of enzymes e.g. cellulases or xylanases, it may be desirable to subject the cell-free liquid (after precipitation of by products) to a gel filtration step on a column which separates proteins in the molecular range $1 \times 10^3$ to $3 \times 10^5$. An example of a suitable material is that commercially available as "Superose 12" (a cross-linked agarose medium).

The Use of the Enzyme

The lignocellulose-containing plant residue to which the present invention may be applied may be straw, sugar cane, grass, or wood and wood waste such as sawdust or pulp. The process of the present invention may be carried out by agitating mechanically disrupted lignocellulose-containing material (for example finely divided, e.g. 1 to 5 mm particle size, lignocellulose material) with an aqueous medium containing the purified enzyme. The temperature is preferably in the range 30 to 37°C. The pH is preferably in the range 6 to 8.

The duration of the treatment will depend upon the degree of lignin breakdown required and may for example be in the range 1 to 2 days.

The invention will now be further illustrated by reference to the following Examples.

Example 1

An aqueous mixture containing 0.5% wt of ball-milled barley straw, 0.01M $KH_2PO_4$, 0.01M $K_2HPO_4$, $(NH_4)_2SO_3$ (0.1g/1), NaCl(0.3g/1), $MgSO_4$(0.1g/1), $CaCO_3$ (0.02g/1), yeast extract (1.0 g/1) and trace elements was inoculated with S. cyaneus and maintained at 37°C for 2 days. It was then filtered to remove _inter alia_ bacterial cells and a concentrated aqueous solution of ammonium sulphate was added to precipitate the enzyme at 50% saturation of ammonium sulphate. This precipitate was dialysed against $H_2O$ for 2 days at 4°C, and then freeze dried. The resulting material was then subjected to gel filtration on Superose 12 (a commercially cross-linked agarose medium available from Pharamacia Ltd) at 20°C in 0.1 M Tris (hydroxymethyl) methylamine at a pH of 7.0, to give a enzyme preparation of increased purity.

Test carried out using the crude cell-free enzyme i.e. the product obtained after filtration but before precipitation of the protein showed that it had a high activity for the decomposition of lignocelluloses.

The presence of lignin solubilising activity was demonstrated by measuring the solubilisation of lignocellulose labelled with carbon-14. The lignocellulose was derived from wheat and was prepared as described by A.J. McCarthy and P. Broda, J. Gen. Microbiol. 130:2905-2913. This material had a specific activity of 3700 dpm per mg of substrate. The labelled lignocellulose was suspended in 20 mM of Tris HCl (pH7.0) containing 0.1% Triton X-100 and was subjected to ultra-sound for 30 s. It was then centrifuged (1500 g (g = acceleration due to gravity)) for 10 min at 4°C. The substrate was washed again before a final dilution to 5 mg/ml. For assay, 50 microlitres of the substrate suspension (corresponding to 0.25 mg) was added to the liquid containing the enzyme (200 microlitres) after precipitation of by-products with ammonium sulphate. The sample was incubated overnight at 37°C and then centrifuged. The supernatant liquid (50 microlitres) was added to 4 ml of scintillation fluid (Optiphase Safe (Trademark) supplied by LKB) and the radioactivity was measured.

Nearly 30% of the carbon-14 present in the labelled lignocellulose was found in the supernatant liquid.

This shows that lignocellulose has been broken down by an enzyme into soluble products.

Example 2

An experiment was carried out as in Example 1 except that the bacterium was grown for 6 days. The proportion of the labelled carbon in the supernatant liquid was about 20%.

Example 3

An experiment was carried out as in Example 1 except that the bacterium was grown for 9 days. The proportion of the carbon-14 in the supernatant liquid was ca.26%.

Example 4

An experiment was carried out as in Example 1 except that the bacterium was Thermomonospora mesophila NCIB 12382. The proportion of the carbon-14 in the supernatant liquid was ca.5%.

Example 5

An experiment was carried out as in Example 4 but with the bacterium grown for 6 days. The proportion of carbon-14 in the supernatant liquid was ca. 10%.

Example 6

An experiment was carried out as in Example 4 except that the bacterium was grown for 9 days. The proportion of carbon-14 in the supernatant liquid was ca. 15%.

Claims:

1. The process for the decomposition of lignocellulose which comprises bringing lignocellulose into contact with an aqueous medium containing a cell-free enzyme having lignin-solubilising activity derived from an actinomycete bacterium.

2. The process according to claim 1 wherein the lignocellulose is brought into contact with the enzyme at a pH in the range 6 to 8.

3. The process according to claim 1 or 2 wherein the temperature is in the range 30° to 45°C.

4. The process according to claim 3 wherein the temperature is in the range 30° to 37°C.

5. The process according to any one of the preceding claims wherein the lignocellulose is maintaining in contact with the enzyme for 1 to 2 days.

6. The process according to any one of the preceding claims wherein the lignocellulose is in mechanically disrupted form.

7. The process according to any one of the preceding claims wherein the aqueous medium containing the enzyme brought into contact with the lignocellulose is substantially free from cellulases and xylanases.

8. A cell-free liquid containing an enzyme having lignin-solubilising activity derived from an actinomycete bacterium.

9. A cell-free liquid according to claim 8 containing a lignin-solubilising preparation derived from Streptomyces cyaneus.

10. A cell-free liquid according to claim 9 derived from Streptomyces cyaneus NCIB 12383.

7

11. A cell-free enzyme having lignin-solubilising activity, substantially free from cellulases and xylanases, and having an apparent molecular weight of about 20,000 by gel permeation, chromatography and an isoelectric point in the range 5 to 5.6.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,A | EP-A-0 188 931 (INSTITUT NATIONAL DE LA RECHERCHE AGRONOMIQUE) * Claims * | 1 | C 12 N 9/02 C 12 P 7/00 |
| A | EP-A-0 094 256 (GENETICS INTERNATIONAL) * Claims * | 1 | |
| A | CHEMICAL ABSTRACTS, vol. 91, no. 21, 19th November 1979, page 353, abstract no. 171565w, Columbus, Ohio, US; M.B. PHELAN et al.: "Isolation of lignocellulose-decomposing actinomycetes and degradation of specifically 14C-lignocelluloses by six selected Streptomyces strains", & CAN. J. MICROBIOL. 1979, 25(11), 1270-6 * Abstract * | 1 | |

**TECHNICAL FIELDS SEARCHED (Int. Cl.4)**

C 12 N

The present search report has been drawn up for all claims

| Place of search | Date of completion of the search | Examiner |
|---|---|---|
| THE HAGUE | 20-07-1987 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82